Europäisches Patentamt

European Patent Office

Office européen des brevets

(19)

(11) Publication number: **0 206 065**
A2

# (12) EUROPEAN PATENT APPLICATION

(21) Application number: 86107789.9

(22) Date of filing: 07.06.86

(51) Int. Cl.⁴: **G 01 N 33/574,** G 01 N 33/577 // C12Q1/68

(30) Priority: 27.06.85 US 749932

(43) Date of publication of application: 30.12.86
Bulletin 86/52

(84) Designated Contracting States: DE FR GB IT

(71) Applicant: **Feramisco, James R., Cold Spring Harbor Laboratory P.O. Box 100, Cold Spring Harbor New York 11724 (US)**
Applicant: **O'Brien, Timothy J., University of Arkansas for Medical Science 4301 W. Markham, Little Rock Arkansas 72205 (US)**

(72) Inventor: **Feramisco, James R., Cold Spring Harbor Laboratory P.O. Box 100, Cold Spring Harbor New York 11724 (US)**
Inventor: **O'Brien, Timothy J., University of Arkansas for Medical Science 4301 W. Markham, Little Rock Arkansas 72205 (US)**

(74) Representative: **Patentanwälte Schulze Horn und Hoffmeister, Goldstrasse 36, D-4400 Münster (DE)**

(54) Method of detecting oncogene and proto-oncogene related proteins in extracellular biological fluids.

(57) A method for detecting or diagnosing the presence of a cancer, a cancerous condition, or a change in susceptibility to cancer which comprises measuring or detecting levels of proto-oncogene or oncogene related proteins in extracellular biological fluid is disclosed. Additionally, kit for use in the method as described are disclosed.

EP 0 206 065 A2

## FIELD OF THE INVENTION

This invention relates to methods of diagnosing and detecting various cancers, changes in cancerous conditions, and cancer susceptibility, by determining levels of proto-oncogene and oncogene related proteins in extracellular biological fluids.

## PRIOR ART

Weinberg, Scientific American 249: 126-142 (1983), provides an overview of the state of the art up to 1983. The reference discusses, e.g., the steps leading to the identification of proto-oncogenes and oncogenes, the RAS group of oncogenes, and point mutations which result in oncogenes.

Wigler, et al, in U.S. Patent Serial No. 595,745, filed April 2, 1984, disclose the N-RAS oncogene, which was derived from a human bladder carcinoma cell line. Wigler et al, in Serial No. 595,744, also filed on April 2, 1984, discloses Ki-RAS oncogene (or, ras$^{Ki}$), derived from human lung carcinoma.

Lane et al, in PNAS 78, 5185-5189 (1981), and Shilo et al, Nature 289: 607-609 (1981), disclose that cells which take up oncogenes become transformed, and will form tumors in mice. Parada et al, Nature 297: 474-479 (1982), Santos et al, Nature 298: 343-347 (1982), and Yuasa et al, Nature 303: 775-779 (1982), all disclose gene point mutation

- 2 -

of p21, which result in altered p21 protein, at either amino acid residue 12 or 61. Marshall et al, in Cancer Survey 3: 183-214 (1984), disclose that residue 12 is most frequently altered.

Furth et al, J. Virol. 43: 294-304 (1982) disclose antibodies which do not discriminate between proto-oncogene and oncogene p21.

Hand et al, PNAS 81: 5227-5231 (1984) disclose monoclonal antibodies which react with p21. The antibodies were not shown to be specific for either oncogene or proto-oncogene p21 protein.

Slamon, et al, Science 224: 256-262 (1984) disclose that various intracellular oncogene and proto-oncogene protein levels are associated with different carcinomas.

## BACKGROUND

The study of cancer and its causes has made remarkable progress in the last decade. Among the most significant of the advances in the field has been the discovery of a genetic basis for the onset of cancer, that is, the discovery of proto-oncogenes and oncogenes.

As is described by Weinberg, Scientific American 249: 126-142 (1983), it was discovered that there are sequences of DNA in cells which have potential to become implicated in cancer. These normal genes are known as proto-oncogenes. Under normal circumstances, these genes may code or express normal proteins which are involved in cellular processes, such as cell growth and growth regulation. As with all genes, these may operate on a continuous, or intermittent bases.

For various reasons, and because of various factors, proto-oncogenes may become transformed into oncogenes. It has been learned that the transformation takes place at the level of single nucleotide bases, or, by "point mutation" of the proto-oncogene. Weinberg, supra. The change in a single nucleotide base causes the resulting protein to differ in one amino acid residue. Remarkably, this difference of one amino acid can change the growth rate of the cell containing it by a factor of anywhere from 10 to 1000.

The past few years have seen the discovery of many proto-oncogenes which, in turn, become transformed into oncogenes. Slamon, supra, e.g., lists various oncogenes, and the tumors associated therewith.

Slamon, et al, supra, disclose the presence or absence of various intracellular oncogene products, when various tumor types are observed. This information is presented in Table I, viz:

- 4 -

## TABLE I

| Type of tumor | Number of tumor | abl | fes | fos | fms | myb | myc | ras$^{Ha}$ | ras$^{Ki}$ | src |
|---|---|---|---|---|---|---|---|---|---|---|
| **Renal malignant** | | | | | | | | | | |
| Renal cell carcinoma | 1 | 0 | 0 | ++ | 0 | 0 | +++ | ++ | ++ | 0 |
| | 2 | 0 | 0 | +++ | 0 | 0 | +++ | ++ | ++ | 0 |
| | 3 | 0 | 0 | +++ | 0 | 0 | +++ | ++ | ++ | 0 |
| | 4 | 0 | 0 | ++ | 0 | 0 | ++ | ++ | ++ | 0 |
| | 5 | 0 | 0 | 0 | 0 | 0 | 0 | 0 | 0 | 0 |
| | 6 | 0 | 0 | ++ | + | 0 | + | + | 0 | 0 |
| | 7 | 0 | 0 | ++ | ++ | 0 | ++ | ++ | 0 | 0 |
| | 8 | 0 | ++ | 0 | ++++ | 0 | ++ | 0 | ++ | 0 |
| | 9 | 0 | + | +++ | ++ | 0 | + | + | 0 | 0 |
| **Gynecologic malignancies** | | | | | | | | | | |
| Ovarian adenocarcinoma | 1 | 0 | 0 | ++++ | 0 | 0 | +++ | ++ | ++ | 0 |
| | 2 | 0 | 0 | ++++ | 0 | 0 | ++++ | +++ | +++ | 0 |
| | 3 | 0 | 0 | ++ | + | 0 | ++ | ++ | + | 0 |
| | 4 | 0 | 0 | ++++ | + | 0 | ++ | + | 0 | 0 |
| | 5 | 0 | 0 | ++++ | + | 0 | + | ++ | + | 0 |
| | 6 | 0 | 0 | ++ | + | 0 | +++ | ++ | 0 | 0 |
| Uterine adenocarcinoma | 1 | 0 | 0 | 0 | 0 | 0 | 0 | 0 | 0 | 0 |
| Germ-cell tumor | 1 | 0 | 0 | ++ | 0 | 0 | ++ | ++ | ++ | 0 |
| **Gastrointestinal malignancies** | | | | | | | | | | |
| Colon adenocarcinoma | 1 | 0 | 0 | +++ | 0 | 0 | ++ | ++ | ++ | 0 |
| | 2 | 0 | 0 | +++ | 0 | 0 | ++ | ++ | ++ | 0 |
| | 3 | 0 | 0 | +++ | 0 | 0 | ++ | ++ | + | 0 |
| | 4 | 0 | 0 | +++ | 0 | 0 | ++ | ++ | + | 0 |
| | 5 | 0 | 0 | +++ | 0 | 0 | +++ | +++ | + | 0 |
| Rectal adenocarcinoma | 1 | 0 | 0 | +++ | 0 | 0 | +++ | ++ | ++ | 0 |
| | 2 | 0 | 0 | +++ | 0 | 0 | 0 | 0 | 0 | 0 |
| | 3 | 0 | 0 | +++ | 0 | 0 | ++ | 0 | | 0 |
| Cecal adenocarcinoma | 1 | 0 | 0 | +++ | + | 0 | + | + | 0 | 0 |
| Small bowel adenocarcinoma | 1 | 0 | 0 | +++ | 0 | 0 | ++ | ++ | ++ | 0 |
| Pancreatic adenocarcinoma | 1 | 0 | 0 | ++ | ++ | 0 | + | ++ | 0 | 0 |
| **Lung malignancies** | | | | | | | | | | |
| Carcinoma of lung | 1 | 0 | ++ | ++ | 0 | 0 | +++ | +++ | +++ | 0 |
| | 2 | 0 | ++ | ++ | 0 | + | ++ | ++ | ++ | 0 |
| | 3 | 0 | + | ++ | ++ | 0 | ++ | ++ | + | 0 |
| | 4 | 0 | + | + | ++ | 0 | + | + | + | 0 |
| **Breast malignancies** | | | | | | | | | | |
| Carcinoma of breast | 1 | 0 | 0 | ++ | 0 | + | ++ | ++ | 0 | 0 |
| | 2 | 0 | ++ | ++ | +++ | ++ | +++ | ++ | + | 0 |
| | 3 | 0 | ++ | ++ | +++ | 0 | +++ | +++ | ++ | 0 |
| | 4 | 0 | + | ++ | +++ | 0 | +++ | ++ | ++ | 0 |
| **Sarcomatous malignancies** | | | | | | | | | | |
| Pelvic sarcoma | 1 | 0 | 0 | ++ | 0 | 0 | + | + | + | 0 |
| Rhabdomyosarcoma | 1 | 0 | 0 | ++ | 0 | 0 | +-+ | ++ | + | 0 |
| **Lymphoreticular malignancies** | | | | | | | | | | |
| Thymoma | 1 | 0 | 0 | 0 | 0 | 0 | + | + | 0 | 0 |
| Hodgkin's lymphoma | 1 | + | + | ++ | ++++ | + | ++ | ++ | + | 0 |
| Non-Hodgkin's lymphoma | 1 | 0 | 0 | ++ | 0 | 0 | + | ++ | 0 | 0 |
| | 2 | 0 | 0 | ++ | 0 | +++ | ++ | ++++ | + | 0 |
| **Hematologic malignancies** | | | | | | | | | | |
| Acute myelogenous leukemia | 1 | 0 | ++ | ++++ | + | ++++ | + | ++ | +++ | 0 |
| | 2 | 0 | ++ | ++ | + | ++++ | + | ++ | +++ | 0 |
| | 3 | 0 | ++ | ++ | 0 | +++ | 0 | + | ++ | 0 |
| | 4 | 0 | ++++ | 0 | 0 | ++++ | + | 0 | + | 0 |
| | 5 | 0 | ++++ | + | 0 | ++++ | +++ | + | + | 0 |
| | 6 | 0 | ++++ | + | 0 | +++ | ++ | + | + | 0 |
| | 7 | + | +++ | + | 0 | ++++ | ++ | + | ++ | 0 |
| Chronic myelogenous leukemia | 1 | ++ | ++ | 0 | 0 | +++ | + | + | + | 0 |
| | 2 | + | +++ | ++ | ++++ | ++ | ++ | ++ | + | ++ |
| Acute lymphocytic leukemia | 1 | 0 | +++ | ++ | 0 | +++ | + | + | ++' | 0 |
| | 2 | 0 | ++ | + | 0 | +++ | ++ | ++ | + | 0 |
| Lymphosarcoma (leukemic phase) | 1 | 0 | 0 | + | 0 | 0 | ++ | + | + | +++ |

- 5 -

Additional oncogenes, which were not expressed in cells of patients carrying the indicated tumors, include $env^A$, $env^B$, mos, rel, sis, and yes oncogenes.

Until the development of the invention as described herein, any and all work involving the detection, labelling, and analysis, either quantitative or qualitative, of oncogene and proto-oncogene proteins, involved analyses of intracellular material. Representative of the state of the art is, e.g., Johnsson et al, PNAS 82: 1721-1725 (March 1985), e.g., which compares platelet derived growth factor to V-sis oncogene product. The oncogene product is obtained by lysing freeze-thawed cells. Papageorge, et al, Mol. Cell. Biol. 4: 23-29 (January 1984), disclose a discovery of a p21 mammalian ras-related protein produced by Saccharomyces cerevisiae. To obtain the protein, cells had to be centrifuged, and extracts were prepared. There was no analysis of the cell culture medium, because, up to the present, it has not been thought possible to detect oncogene and proto-oncogene proteins in extracellular materials.

While it may appear routine to expect that intracellular proteins, such as proto-oncogene and oncogene related proteins may be found in extracellular fluids, such is not the case. Many intracellular proteins never leave the cell, because of their association with various intracellular processes. In the case of oncogene related proteins, this is especially true. As has been pointed out,

- 6 -

supra, the normal intracellular proteins which are transformed into oncogene proteins, are thought to be associated, e.g., with cell growth processes, as well as intracellular regulation. There would, therefore, be no expectation that these proteins could be found outside of the cell.

The new, and surprising discovery that oncogene and proto-oncogene related proteins are found in extracellular fluids is important and useful to the field of cancer diagnosis. The proto-oncogene and oncogene proteins have been associated with cancer of various types. Detection of the protein molecules in extracellular fluids, such as serum, and blood, and exudates such as urine, saliva, sweat, sputum, and the like, will indicate not only that cancer is present, but may be used, e.g., to determine that the chances of an individual patient developing cancer may increase or decrease. For example, it is generally known that exposure to certain carcinogens sometimes leads to increased rates of cancer. Examination of individuals before, and after exposure to a carcinogen, such as asbestos, would now enable one to determine if increased oncogene or proto-oncogene related proteins are present, and hence if the risk of developing cancer has increased. Further, an individual's body fluids may be assayed during cancer therapy to determine if the protein levels have decreased, and hence if the cancer has receded. Both of these aspects are a marked improvement over the usual surgical biopsy, which must be performed to determine presence or recession of cancer.

The invention also allows for determination of the "phenotype", or category, of cancer of a patient diagnosed as carcinogenic. Many types of cancer are associated with one or more particular oncogenes (Slamon, supra), and determination of what protein(s) is or are present, using, e.g., antibody assays, allows one to determine the particular type of cancer, and then a method of therapy for treating the patient.

The fact that the proteins are found in extracellular fluids means that cell samples of the individual patient need no longer be taken, and the concomitant involved process of cell lysis, fractionation, etc., may be avoided. It is standard practice in medical examinations to administer tests which involve analysis of body fluids (e.g., blood tests, urine tests). Samples taken for old tests may now also be used to determine presence and levels of oncogene and proto-oncogene proteins. The patient does not have to undergo biopsies, radioactive assay series, and so forth. Instead, analysis of the protein content of the individual's samples of serum, blood, urine, etc., may be used to determine the presence of cancer, changes in susceptibility to cancer, or in a cancerous condition.

BRIEF DESCRIPTION OF THE FIGURES

Figure 1 gives a standard profile for the fractionation studies described herein.  The standard profile represents the conditions used for all fractionations described herein.

Figure 2 is a fractionation profile for one of the six samples used, and shows the twenty six different fractions obtained.  It is noted that albumin is the eleventh fraction.

Figures 3-8 show the "dot blotting" results (Column X) as compared to controls, for the serum samples described herein.

Figure 9 shows the results of SDS gel studies on pooled fractions of the serum samples, which showed strong possibility of containing RAS protein.

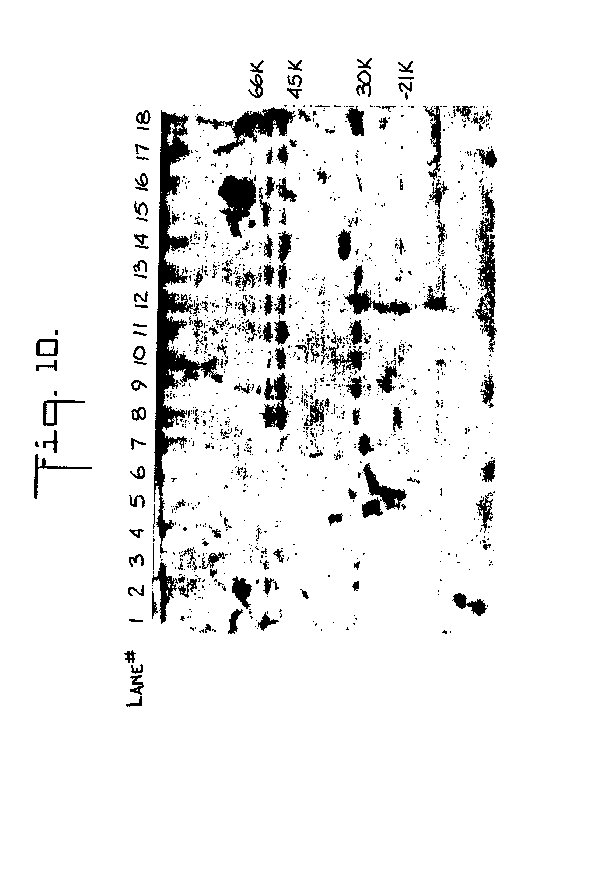

Figure 10 is figure 9 after treatment to remove albumin.

DETAILED DESCRIPTION OF PREFERRED EMBODIMENTS

EXAMPLE 1

I.  Fractionation of Serum Samples

Serum samples were collected from different sources (fetal serum; 12 week pregnancy serum; 40 week pregnancy serum; 16 week amniotic fluid; 40 week amniotic fluid; and serum from a patient with uterine cancer).  These samples were then fractionated using an FPLC (fast protein liquid chromatography) system, including anion exchange (mono Q), and salt gradient elution (0.5 ml of serum sample used).

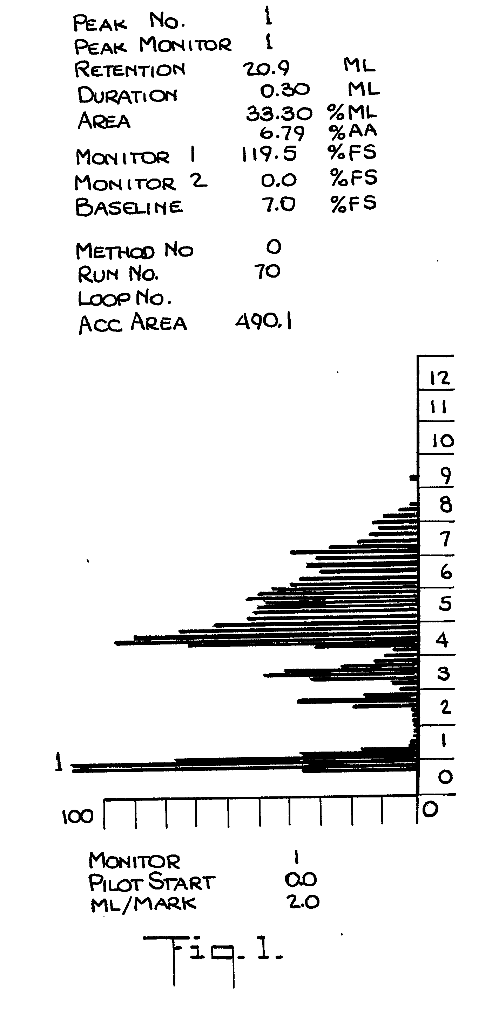

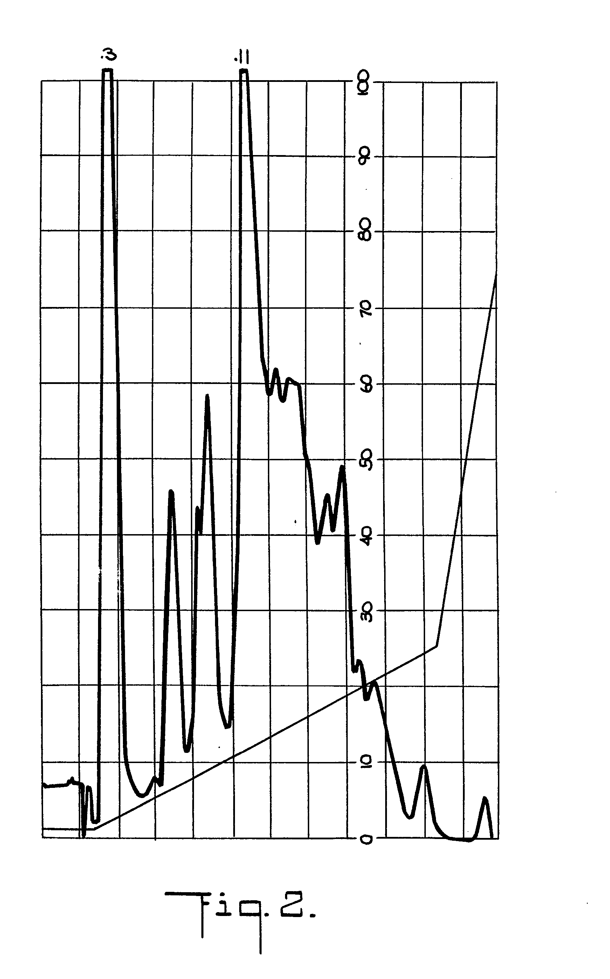

Figure 1 shows the standard profile under the conditions used.  Figure 2 shows the profile for one of the samples (pregnancy serum -   weeks).  Only one profile is given because, with minor variations, all profiles were alike.

Twenty-six separate fractions of each serum sample were thus collected.  These were analyzed for detection of RAS or RAS related proteins.

II.   Dot-Blot Immunoassay

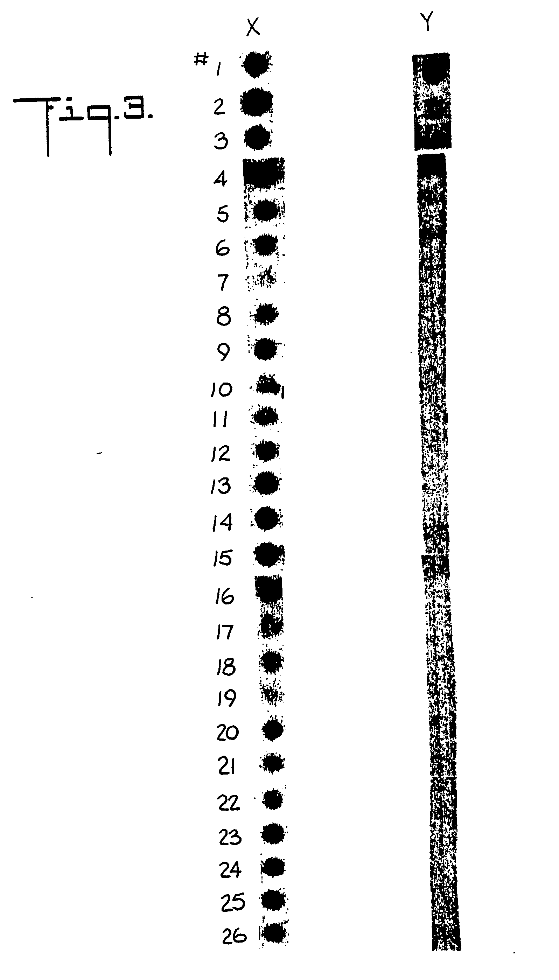

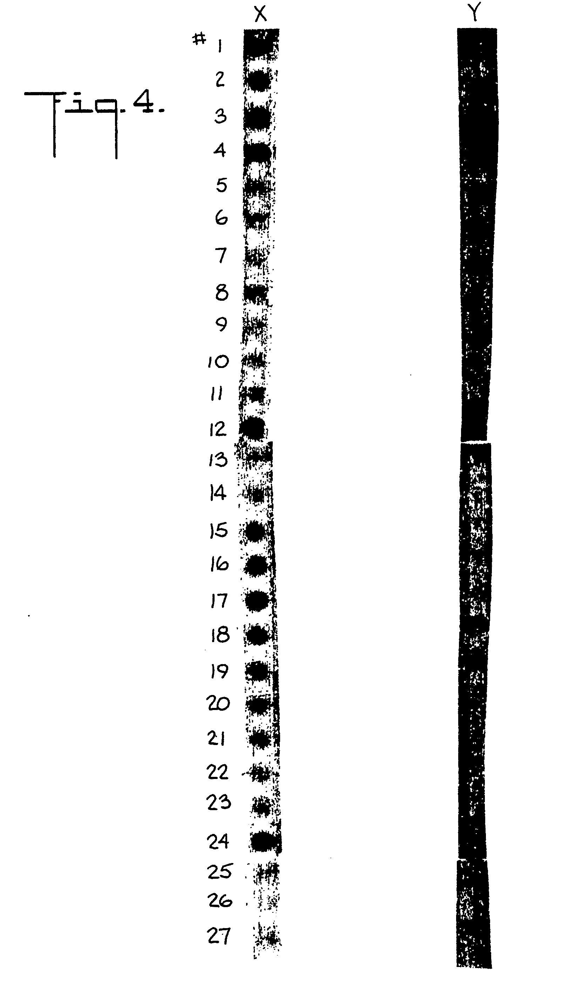

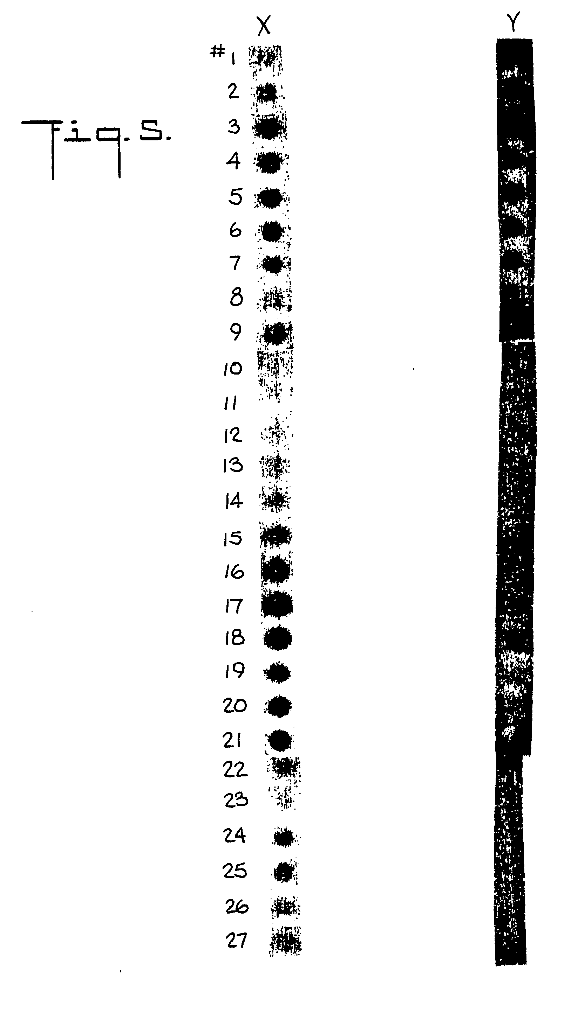

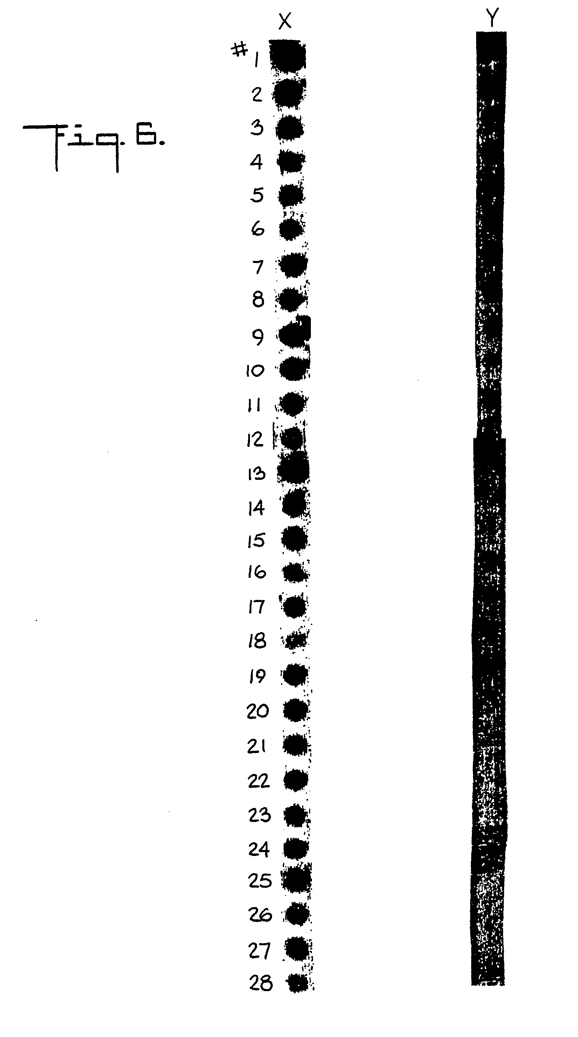

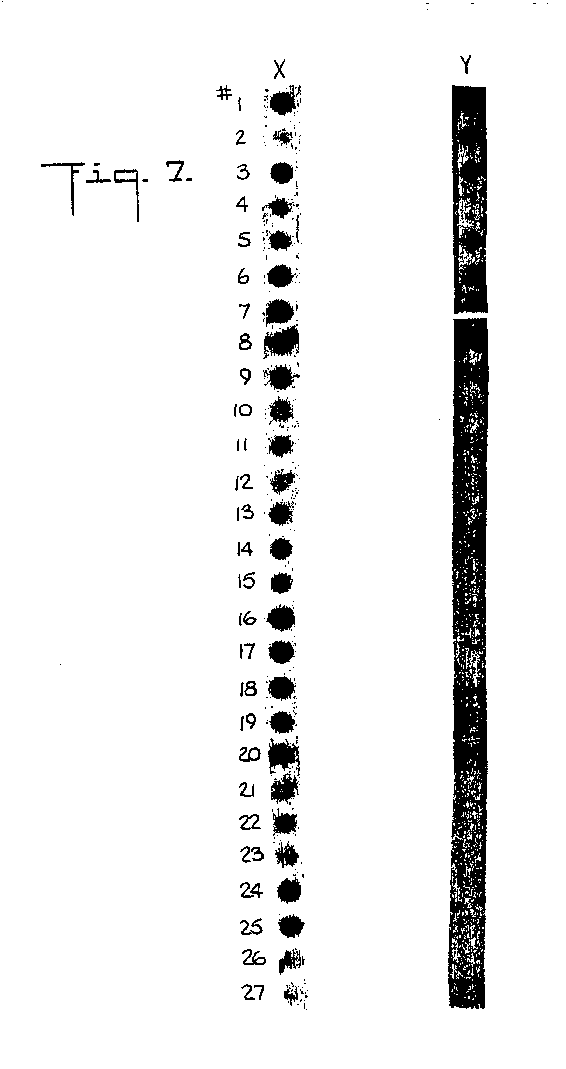

The twenty-six tractions obtained from each sample were "dot blotted" for immunoassay.  In this method, aliquots of each sample (150 ul) were "dot blotted" to nitrocellulose filters.  The filters with the blotted samples are then exposed to rabbit A anti RAS antibody (dilution 1:100), and incubated under conditions favoring the binding of this antibody to any epitopes present in the nitrocellulose filter. Following the first incubation, a second sample of antibody, i.e., $^{125}$I-labelled goat-antirabbit IgG antibody is contacted to the filter, and incubated. These second antibodies will bind to any antibodies from the first sample which have become bound during the first incubation. The filters are then exposed to X-ray film, and the results are shown in Column X of Figures 3-8.

III.  Results

After the two incubations and the X-ray film exposure described in II, supra, the film is developed and compared to a control (i.e., a sample incubated with normal, non-immunized rabbit serum, and then $^{125}$I labelled second antibody.

Analysis of the film shows some non-specific binding in the first few fractions, and this is probably due to human IgG. As will be seen, particularly in Figures 3, 4, and 8 (pregnancy sera and tumor serum), there is specific binding for RAS protein especially in the latter fractions (especially 12-18, and 23-25). These are all post albumin fractions (i.e., albumin is fraction 11).

In order to further confirm the presence of RAS type proteins in the samples, fractions were pooled where dot blot assay showed positive results (for example, samples

- 11 -

15-18 of pregnancy serum, samples 13-16 for tumor serum).
Control sera for normal males and females were also used.

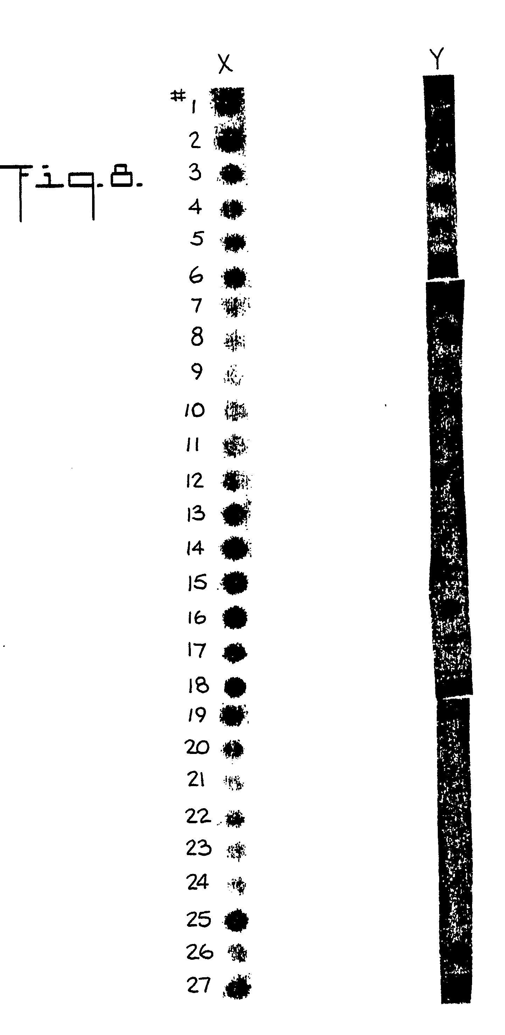

The pooled samples were concentrated, and run on
sodium duodecyl sulfate (SDS) polyacrylamide gel, in order
to separate the proteins on the basis of size.  Separated
proteins in the gels were then transferred to filters by
electrophoretic blotting, and the filters were exposed,
first to rabbit A.  Anti RAS antibodies were incubated, and
then to $^{125}$I protein A or $^{125}$I goat anti rabbit anti RAS
IgG, again followed by incubation.  Exposure to X-ray film
produced the results shown in Figure 8.

Lanes 13-18 show strongly the presence of a 21K
protein.  These lanes represent 16 week pregnancy serum (13
and 14), 40 week pregnancy serum (15 and 16), and tumor
serum (17 and 18).  Lames 5-8 are control sera.

Lanes 13-18 also show strongly a 14K protein,
which is probably a breakdown product of 21K protein.  The
higher molecular weight bands are thought to represent
binding of the $^{125}$I antibody, described supra, to human
immunoglobulin.  Removal of the immunoglobulin eliminates
these bands.  A 30K protein band is noticeable, particularly
in lanes 3 and 4.  These lanes represent tumor patient
serum.

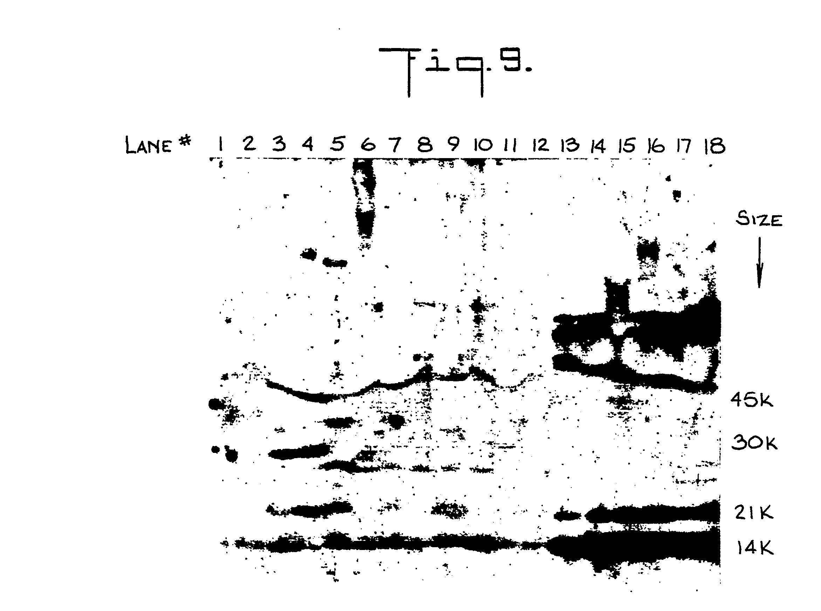

Albumin was then removed from all the samples, as
it distorts the gels.  Tests were run, again, and these
results are shown in Figure 9.

- 12 -

Figure 9 shows that the drastic treatment to the sera has probably resulted in removal of all 21K protein. A band of approximately 31K remains, especially in lanes 15 and 18 (ovarian cancer patient, with extensive disease). Lane 14, a patient with squamous cell carcinoma of the cervix, shows higher weight proteins, while lane 7, a patient with cancer tumors of unknown origin, shows lower weight proteins. Additional tumor sera show dense labelling also.

Exposure of these sera to agarose protein A removes higher weight components (e.g., 50-60K), but not the 31K protein, which is probably being recognized by the RAS antibody.

It will be appreciated by one skilled in the art that various other embodiments of this invention are possible. For example, it is known that in patients with RAS related cancer, the normal immune response will often result in the presence of antibodies in biological fluids of the patient. The method, as heretofore described, would allow one to detect the presence of these antibodies in patients.

### EXAMPLE 2

The steps set forth in Example 1 are employed for analysis of blood, plasma, urine, and biological exudates, such as saliva, sputum and urine.

- 13 -

## EXAMPLE 3

The steps set forth in Example 1 are employed to analyze an extracellular biological fluid for the presence of abl related proteins.

## EXAMPLE 4

The steps set forth in Example 1 are employed to analyze an extracellular biological fluid for the presence of fes related proteins.

## EXAMPLE 5

The steps set forth in Example 1 are employed to analyze an extracellular biological fluid for the presence of fos related proteins.

## EXAMPLE 6

The steps set forth in Example 1 are employed to analyze an extracellular biological fluid for the presence of fms related proteins.

## EXAMPLE 7

The steps set forth in Example 1 are employed to analyze an extracellular biological fluid for the presence of myb related proteins.

- 14 -

## EXAMPLE 8

The steps set forth in Example 1 are employed to analyze an extracellular biological fluid for the presence of _myc_ related proteins.

## EXAMPLE 9

The steps set forth in Example 1 are employed to analyze an extracellular biological fluid for the presence of $ras^{Ha}$ related proteins.

## EXAMPLE 10

The steps set forth in Example 1 are employed to analyze an extracellular biological fluid for the presence of $ras^{Ki}$ related proteins.

## EXAMPLE 11

The steps set forth in Example 1 are employed to analyze an extracellular biological fluid for the presence of _src_ related proteins.

## EXAMPLE 12

The steps set forth in Example 1 are employed to analyze an extracellular biological fluid for the presence of $env^{A}$ related proteins.

EXAMPLE 13

The steps set forth in Example 1 are employed to analyze an extracellular biological fluid for the presence of $\underline{env}^B$ related proteins.

EXAMPLE 14

The steps set forth in Example 1 are employed to analyze an extracellular biological fluid for the presence of $\underline{mos}$ related proteins.

EXAMPLE 15

The steps set forth in Example 1 are employed to analyze an extracellular biological fluid for the presence of $\underline{rel}$ related proteins.

EXAMPLE 16

The steps set forth in Example 1 are employed to analyze an extracellular biological fluid for the presence of $\underline{sis}$ related proteins.

EXAMPLE 17

The steps set forth in Example 1 are employed to analyze an extracellular biological fluid for the presence of $\underline{yes}$ related proteins.

- 16 -

## EXAMPLE 18

An individual is diagnosed as having cancer, but the particular type of cancer has not been determined. A sample of serum is assayed with a panel of monoclonal antibodies to abl, fes, fos, fms, myb, myc, $ras^{Ha}$, $ras^{Ki}$, and src related oncogene proteins. Positive results are found for the fos, myc, $ras^{Ha}$ and $ras^{Ki}$ mAbs. While all of these titers are high, reaction with fos is extremely high. Based on this, and other observations, the individual is diagnosed as having a gastrointestinal related carcinoma.

## EXAMPLE 19

An individual who has suffered from leukemia and has undergone chemotherapy for a period of time is examined for presence of fes and myb oncogene proteins in his blood. The blood assay indicates very low levels of fes and myb oncogene proteins, indicating that the cancer is currently in remission.

One skilled in the art will recognize that there are other apparent aspects of the invention as well. For example, haptens, labelled enzymes, and the like, may be used to detect the presence of the oncogene and proto-oncogene related proteins.

The terms and expression which have been employed are used as terms of description and not of limitation, and there is no intention in the use of such terms and expressions of excluding any equivalents of the features shown and escribed or portions thereof, it being recognized that various modifications are possible within the scope of the invention.

Claims:

1. A method for detecting or diagnosing the presence of a cancer, a cancerous condition, or a change in susceptibility to cancer comprising detecting the presence of changed levels of proto-oncogene or oncogene proteins in extracellular biological fluid of a patient and comparing said levels to a basal level of said proteins wherein said basal level is characteristic of normal patients.

2. A method as in claim 1, wherein said cancer is a renal carcinoma, said cancerous condition is a renal cancer condition, or said change in susceptibility is to renal carcinoma, and said proto-oncogene or oncogene protein is a protein expressed by at least one oncogene selected from the group consisting of fes, fos, myc, fms, $ras^{Ha}$, and $ras^{Ki}$.

3.  A method as in claim 1, wherein said cancer is a gynecologic cancer, said cancerous condition is a gynecological cancer related condition, or said change in susceptibility is to gynecological cancer, and said proto-oncogene or oncogene protein is a protein expressed by at least one oncogene selected from the group consisting of fos, myc, $ras^{Ha}$, $ras^{Ki}$, and fms.

4.  A method as in claim 1, wherein said cancer is a gastrointestinal cancer, said cancerous condition is a gastrointestinal cancer related condition, or said change in susceptibility is to gastrointestinal cancer, and said proto-oncogene or oncogene protein is a protein expressed by at least one oncogene selected from the group consisting of fos, myc, $ras^{Ha}$, $ras^{Ki}$, and fms.

5.  A method as in claim 1, wherein said cancer is a lung cancer, said cancerous condition is a lung cancer related condition or said change in susceptibility is to lung cancer, and said proto-oncogene or oncogene protein is a protein expressed by at least one oncogene selected from the group consisting of fes, fos, myc, $ras^{Ha}$, $ras^{Ki}$, and fms.

- 20 -

6.  A method as in claim 1, wherein said cancer is a breast cancer, said cancerous condition is a breast cancer related condition, or said change in susceptibility is to breast cancer, and said proto-oncogene or oncogene protein is a protein expressed by at least one oncogene selected from the group consisting of fes, fos, fms, myb, myc, ras$^{Ha}$, and ras$^{Ki}$.

7.  A method as in claim 1, wherein said cancer is a sarcoma, said cancerous condition is a sarcomatous related condition, or said change in susceptibility is to sarcoma, and said proto-oncogene or oncogene protein is a protein expressed by at least one oncogene selected from the group consisting of fos, myc, ras$^{Ha}$, and ras$^{Ki}$.

8.  A method as in claim 1, wherein said cancer is a lymphoreticular cancer, said cancerous condition is a lymphoreticular cancer related condition, or said change in susceptibility is to lymphoreticular cancer, and said proto-oncogene or oncogene protein is a protein expressed by at least one oncogene selected from the group abl, fes, fos, fms, myb, myc, ras$^{Ha}$, and ras$^{Ki}$.

9. A method as in claim 1, wherein said cancer is a hematologically related cancer, said cancerous condition is a hematological cancer related condition, or said change in susceptibility is to hematologically related cancer, and said proto-oncogene or oncogene protein is expressed by at least one oncogene selected from the group consisting of abl, fes, fos, fms, myb, myc, $ras^{Ha}$, $ras^{Ki}$, and src.

10. A method as in claim 1, wherein said proto-oncogene or oncogene protein is expressed by at least one oncogene selected from the group consisting of abl, fes, fos, fms, myb, myc, $ras^{Ha}$, $ras^{Ki}$, and src.

11. A method as in claim 2 or 3 wherein said oncogene is selected from the group consisting of fos, myc, and $ras^{Ha}$.

12. A method as in claim 4, wherein said oncogene is selected from the group consisting of fos, myc, $ras^{Ha}$, and $ras^{Ki}$.

13. A method as in claim 5, wherein said oncogene is selected from the group consisting of fes, fos, myc, $ras^{Ha}$ and $ras^{Ki}$.

14. A method as in claim 6, wherein said oncogene is selected from the group consisting of fos, fms, myb, $ras^{Ha}$, and $ras^{Ki}$.

15. A method as in claim 7 or 8, wherein said oncogene is selected from the group consisting of fos, myc, and $ras^{Ha}$.

16. A method as in claim 9, wherein said oncogene is selected from the group consisting of fes, fos, myb, myc, $ras^{Ha}$, and $ras^{Ki}$.

17. A method as in claim 1, wherein said extracellular biological fluid is serum, blood, or a biological exudate.

18. A method as in claim 1, wherein said substance is an antibody, especially a monoclonal antibody.

19. A kit for use in detecting or diagnosing the presence of a cancer, or cancerous condition or change in susceptibility to cancer comprising a substance which specifically identifies the presence of a proto-oncogene or oncogene related protein.

20. A kit as in claim 19, wherein said proto-oncogene or oncogene related protein is expressed by an oncogene selected from the group consisting of abl fes, fos, fms, myb, myc, $ras^{Ha}$, $ras^{Ki}$, and src.

21. A kit as in claim 19, wherein said substance is an antibody, especially a monoclonal antibody.

22. A method of phenotyping cancer comprising contacting extracellular biological fluid with at least one substance which specifically binds to a proto-oncogene or oncogene related protein associated with a particular form of cancer, and identifying the type of cancer present by detecting said protein.

- 23 -

0206065

23. A kit for phenotyping cancer comprising at least one substance which specifically binds to a proto-oncogene or oncogene protein associated with a particular cancer.

24. A method or a kit as in cleim 22 or 23 wherein said substance is an antibody, especially a monoclonal antibody.

- 24 -

PEAK No.            1
PEAK MONITOR       1
RETENTION        20.9      ML
DURATION          0.30     ML
AREA             33.30    %ML
                  6.79    %AA
MONITOR 1        119.5    %FS
MONITOR 2          0.0    %FS
BASELINE           7.0    %FS

METHOD No          O
RUN No.           70
LOOP No.
ACC AREA         490.1

MONITOR          1
PILOT START      0.0
ML/MARK          2.0

Fig. 1.

Fig. 2.

Fig.3.

Fig.4.

X

#  1
2
3
4
5
6
7
8
9
10
11
12
13
14
15
16
17
18
19
20
21
22
23
24
25
26
27

Y

Fig.5.

Fig. 6.

Fig. 7.

Fig.8.

Fig. 9.

Fig. 10.